Europäisches Patentamt

European Patent Office

Office européen des brevets

(19)

(11) EP 1 079 803 B1

(12) EUROPEAN PATENT SPECIFICATION

(45) Date of publication and mention
of the grant of the patent:
**18.08.2004 Bulletin 2004/34**

(51) Int Cl.⁷: **A61K 9/08**, A61K 9/19,
A61K 47/02, A61K 47/12,
A61K 47/26, A61K 38/29

(21) Application number: **99917715.7**

(22) Date of filing: **26.04.1999**

(86) International application number:
**PCT/CA1999/000376**

(87) International publication number:
**WO 1999/055353 (04.11.1999 Gazette 1999/44)**

(54) **PROTEIN FORMULATIONS**

PROTEINHALTIGE ARZNEIMITTEL

FORMULATIONS PROTEINIQUES

(84) Designated Contracting States:
**AT BE CH CY DE DK ES FI FR GB GR IE IT LI LU
MC NL PT SE**
Designated Extension States:
**AL LT LV MK RO SI**

(30) Priority: **28.04.1998 SE 9801495**

(43) Date of publication of application:
**07.03.2001 Bulletin 2001/10**

(73) Proprietor: **NPS Allelix Corp.
Mississauga Ontario, L4V 1V7 (CA)**

(72) Inventors:
• **BILLGER, Martin
S-431 83 Molndal (SE)**

• **BRULLS, Mikael
S-431 83 Molndal (SE)**

(74) Representative: **Wichmann, Hendrik, Dr.
Patentanwälte
Isenbruck Bösl Hörschler Wichmann Huhn
Postfach 86 08 80
81635 München (DE)**

(56) References cited:
| | |
|---|---|
| **EP-A- 0 619 119** | **WO-A-91/06564** |
| **WO-A-95/17207** | **WO-A-97/14429** |
| **WO-A-99/07340** | **US-A- 5 563 122** |

**Description**

TECHNICAL FIELD

The present invention relates to pharmaceutical formulations comprising human parathyroid hormone (PTH). useful for the treatment of bone related disorders such as osteoporosis.

BACKGROUND ART

*Parathyroid hormone*

Human parathyroid hormone is an 84 amino acid protein involved in calcium and phosphorus homeostasis and control of bone growth and density. Equivalent terms for parathyroid hormone is PTH and the less frequently used parathyrin and parathormone.

Human PTH may be obtained through tissue extraction, from peptide synthesis or from genetically engineered yeast, bacterial or mammalian cell hosts. Essentially pure human PTH is disclosed in US 5,208,041. Recombinant production of PTH in *E. coli* is disclosed e.g. in US 5.223.407, US 5,646,015 and US 5,629,205. Production of recombinant human PTH in yeast is disclosed in EP-B-0383751. Synthetic human PTH is commercially available from Bachem Inc., Bubendorf, Switzerland.

In mammals, the balance between bone formation, associated with the activity of osteoblasts, on one hand, and bone loss, associated with the activity of osteoclasts. on the other hand, is disturbed in several bone affecting diseases, such as osteoporosis. Parathyroid hormone has been shown to have a potential therapeutic role in osteoporosis. The anabolic actions of parathyroid hormone on bone are reviewed by Dempster, D.W. *et al.* in Endocrine reviews vol. 14(6), 690-709, 1993.

For many proteins it is common that increasing the protein concentration increases the propensity for protein aggregation and precipitation. either specific or non-specific. Such reactions may be rapid. or may proceed slowly. so the aggregation may not always be clearly evident. In addition. high protein concentrations may reveal small but significant autocatalytic activities. that increases protein degradation. Regardless, aggregation and precipitation affect not only the available protein drug concentration but also the physical and biopharmaceutical properties of the formulation. From a pharmaceutical point of view, this is highly undesirable. A common prejudice is therefore to keep protein concentrations low.

It is known that PTH forms aggregates when the concentration is increased. One strategy to overcome this problem is to modify pH to either very low or very high values. However, this often stimulates chemical degradation of the protein, and further adds to discomfort during administration, e.g. subcutaneous injection.

Dilute formulations necessitates larger volumes to obtain the same dose in . for example. parenteral formulations. Not only does this lead to larger syringes and packages, but also to discomfort during administration and higher production costs. From this reasoning. it follows that a protein formulation should be as concentrated as possible. Furthermore. the development of a non-parenteral dosage form. benefits that the protein concentration is as high as possible. Hence. there is a need for a formulation that allow for a high protein concentration without aggregation.

*PTH formulations*

Unlike other proteins that have been successfully formulated, PTH is particularly sensitive to various forms of degradation. For example, oxidation can occur at methionine residues at positions 8 and 18. giving rise to the oxidized PTH species ox-M(8)-PTH and ox-M(18)-PTH, while deamidation can occur at asparagine in position 16, giving rise to d16-PTH. The polypeptide chain becomes truncated by breakage of peptide bonds, both at the N- and C-terminals. Furthermore. PTH may also be adsorbed to surfaces, form unspecific aggregates and/or precipitate, thus reducing the available concentration of the drug. All these degradation reactions. and combinations thereof. leads to partial or complete loss of PTH bioactivity. A formulation of PTH must therefore prevent these degradation reactions.

WO 95/17207 (Holthuis et al.) discloses a PTH preparation comprising an excipient. e.g. mannitol. that colyophilises with PTH to yield an amorphous cake. and a non-volatile buffering agent, e.g. a citrate source. According to this disclosure, PTH is desirably incorporated in an aqueous solution in a concentration range from 25 to 250 μg/ml. preferably 50 to 150 μg/ml.

US 5,563,122 (Endo et al.) discloses a lyophilized composition comprising PTH(1-34), sodium chloride and a sugar. When lyophilized samples of PTH(1-34) were stored at +40°C for 3 months, it was found that preparations containing combinations of sodium chloride and sugar were more stable than control preparations which contained sodium chloride alone or sugar alone. In these experiments. each sample contained 36 μg PTH( 1-34) together with 5 to 10 mg sugar and 0.1 to 1 mg NaCl.

BRIEF DESCRIPTION OF THE DRAWINGS

Fig. 1
PTH stability at different protein concentrations.

Fig. 2
PTH stability at different pH values.

Fig. 3
PTH stability in the presence of NaCl.

DISCLOSURE OF THE INVENTION

Contrary to the established knowledge. it has been found that relatively high concentrations of PTH can be used in a pharmaceutically acceptable formulation. The formulation may be in liquid form. but may also be lyophilized. and reconstituted prior to either single or multiple administrations. The formulation reduce discomfort during administration due to a small injection volume. while the formulation also has a pH and buffer capacity that reduce pain upon administration. The use of high PTH concentrations also offers the possibility to develop non-parenteral dosage forms, such as nasal, inhalable and oral formulations, or transdermal formulations. The possibility of a high PTH concentration also allows for a safe and economical multidose formulation, that is suitable for e.g. self-administration.

Consequently, the present invention provides in a first aspect a pharmaceutical formulation comprising human parathyroid hormone (PTH) at a concentration of or above 0.3 mg/ml, such as from 0.3 to 10 mg/ml: a pharmaceutically acceptable buffer having a pH from 4 to 6; and at least one tonicity modifier.

The term "parathyroid hormone" (PTH) encompasses naturally occurring human PTH, as well as synthetic or recombinant PTH (rPTH).

Further, the term "parathyroid hormone" encompasses full-length PTH(1-84) as well as PTH fragments. It will thus be understood that fragments of PTH variants, in amounts giving equivalent biological activity to PTH(1-84), can be incorporated in the formulations according to the invention, if desired. Fragments of PTH incorporate at least the amino acid residues of PTH necessary for a biological activity similar to that of intact PTH. Examples of such fragments are PTH (1-31), PTH(1-34). PTH(1-36), PTH(1-37), PTH(1-38). PTH(1-41), PTH(28-48) and PTH(25-39).

The term "parathyroid hormone" also encompasses variants and functional analogues of PTH. The present invention thus includes pharmaceutical formulations comprising such PTH variants and functional analogues, carrying modifications like substitutions, deletions, insertions. inversions or cyclizations, but nevertheless having substantially the biological activities of parathyroid hormone. Stability-enhanced variants of PTH are known in the art from e.g. WO 92/11286 and WO 93/20203. Variants of PTH can e.g. incorporate amino acid substitutions that improve PTH stability and half-life, such as the replacement of methionine residues at positions 8 and/or 18, and replacement of asparagine at position 16. Cyclized PTH analogues are disclosed in e.g. WO 98/05683.

In this context, the term "biologically active" should be understood as eliciting a sufficient response in a bioassay for PTH activity, such as the rat osteosarcoma cell-based assay for PTH-stimulated adenylate cyclase production (see Rodan et al. (1983) J. Clin. Invest. 72, 1511; and Rabbani et al. (1988) Endocrinol. 123, 2709).

The PTH to be used in the pharmaceutical formulations according to the invention is preferably recombinant human PTH. such as full-length recombinant human PTH.

In preferred forms of the invention, the concentration of the said human parathyroid hormone can be 0.3 - 5 mg/ml or 0.3 - 3 mg/ml; or above 1 mg/ml, such as 1 - 10 mg/ml. I - 5 mg/ml; 1 - 3 mg/ml; or 1 - 2 mg/ml.

The said pharmaceutically acceptable buffer can e.g. be an acetate, a citrate, a phosphate or a carbonate buffer. Preferably, the buffer is a citrate buffer at a concentration from 5 to 20 mM. The said pharmaceutically acceptable buffer has preferably a pH between 5 and 6, most preferably around 5.5.

The said tonicity modifier can e.g. be sorbitol, glycerol, sucrose, or, preferably, sodium chloride and/or mannitol.

In a preferred form of the invention. the PTH formulation can comprise 1 to 3 mg/ml parathyroid hormone, 2 to 5 mg/ml NaCl, 20 to 50 mg/ml mannitol. 5 to 10 mM citrate buffer at a pH between 4 and 6, and optionally a preservative. such as benzyl alcohol or m-cresol.

The pharmaceutical formulations according to the invention are useful in the in the treatment or prevention of bone disorders. in particular osteoporosis.

In a further aspect, the present invention provides a process for the preparation of a pharmaceutical formulation as described above, said process comprising dissolving human parathyroid hormone, to a concentration from 0.3 to 10 mg/ml, and at least one tonicity modifier, in a pharmaceutically acceptable buffer having a pH between 4 and 6.

In another aspect, the invention provides the use of parathyroid hormone, at a concentration from 0.3 to 10 mg/ml. in the manufacture of a pharmaceutical formulation for the treatment or prevention of bone disorders, in particular osteoporosis, said pharmaceutical formulation in addition comprising a pharmaceutically acceptable buffer having a pH between 4 and 6, and at least one tonicity modifier.

EXPERIMENTAL METHODS

The full length human PTH(1-84) used in the formulation studies was produced and excreted from a strain of *Escherichia coli* by known methods (see e.g. US 5,223,407, US 5,646.015 and US 5,629,205). Briefly. the human parathyroid hormone gene on plasmid pJT42 was fused with *E. coli* outer membrane protein A (*ompA*) secretion signal DNA. Also present on the plasmid is a lactose repressor gene (*lacIq*). Translation of the *ompA-rhPTH* mRNA and further processing by endogenous peptidase resulted in the production of mature 84 amino acid human parathyroid hormone which was harvested from the bacterial culture broth.

The recombinant expression was followed by purification of PTH to a preparation essentially free of contaminants. The purification process, which involved methods known in the art (see e.g. US 5,208,041). involved cell separation. filtration, ultrafiltration. ion exchange chromatography, hydrophobic interaction chromatography, preparative reverse phase HPLC, and a second ion exchange chromatography followed by desalting to yield a liquid bulk. The resulting preparation had typically a purity of 95%, or better, as assayed by reversed-phase high performance liquid chromatography (RP-HPLC) and sodium dodecyl sulphate polyacrylamide electrophoresis (SDS-PAGE).

Solutions of PTH were analyzed by RP-HPLC to assess concentration of PTH, purity and formation of oxidized PTH, using trifluoracetic acid and acetonitrile in the mobile phase. Deamidated PTH was determined by cationic exchange HPLC, using a gradient of $K^+$. Retention times for and amounts of PTH, ox-M(8)-PTH, ox-M(18)-PTH and d16-PTH were determined using the appropriate reference standards.

The temperature effect on a rate for a process may be described by the term $Q_{10}$:

$$Q_{10} = \left(\frac{R_2}{R_1}\right)^{\frac{10}{T_2 - T_1}}$$

where $R_1$ and $R_2$ are the observed rates at temperatures $T_1$ and $T_2$, respectively (*cf* Chang, R: Physical chemistry with applications to biological systems. New York, Macmillan Publishing Co., Inc., 1977; Schmidt-Nielsen K. Animal Physiology: Adaptation and environment. 3rd ed. Cambridge, Cambridge University Press. 1983). If $Q_{10} = 2$, then the rate doubles whenever the temperature is increased by 10 degrees; if $Q_{10} = 3$, then the rate triples every 10 degrees. Given that the activation energy is fairly constant for a temperature interval, most reactions and processes have a $Q_{10}$ between 2 and 3.

Aggregation and precipitation was determined by visual inspection of the vials against a white or black background, and by comparison to a suitable reference solution. Bioactivity of PTH was measured using the rat osteosarcoma cell (UMR 106)-based assay of PTH-stimulated adenylate cyclase production (Rodan *et al*., J. Clin. Invest.. 1983. 72:1511; Rabbiani *et al..* Endocrinol., 1988, 123:2709).

EXAMPLE 1 - Effect of protein concentration on PTH stability

Different concentrations of PTH were formulated in 10 mM citrate buffer. In one series of experiments. aliquots of the different formulations were lyophilized and stored at +37°C to +40°C. In another series, aliquots were stored as a liquid at +4°C. At various time points (at least three), the PTH purity were determined by rp-HPLC. Results were expressed as area-%. The data for each time series, representing different concentrations of PTH, were then normalized, by setting the PTH purity at time zero to 100%, to allow comparisons between the series. The rate of PTH loss. expressed as % per month. were calculated from the slope of the linear regression line of % PTH *Vs*. Time, using the formula

$$b = \frac{n\sum xy - \left(\sum x\right)\left(\sum y\right)}{n\sum x^2 - \left(\sum x\right)^2} \quad,$$

where *x* corresponds to time (months) and *y* corresponds to % PTH purity remaining from start.

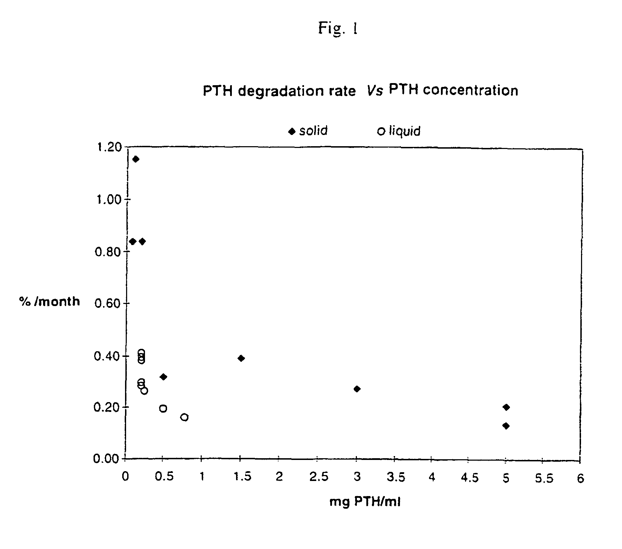

As shown in Fig. 1 and in Tables I and II, the stability of PTH is markedly increased, when the PTH concentration is increased, both for lyophilized (Table I) and liquid (Table II) formulations. Stability is superior at PTH concentrations above 0.3 mg/ml, both for liquid and lyophilized formulations.

EXAMPLE 2 - pH Stability

Six different formulations were prepared consisting of PTH (0.2 mg/ml). sodium citrate/citric acid (10 mM) at pH 4, 4.5, 5, 5.5, 6 and 6.5, respectively, and mannitol (50 mg/ml). Aliquots (1 ml) were sealed in 2 ml glass vials under nitrogen and analyzed for purity of PTH by RP-HPLC.

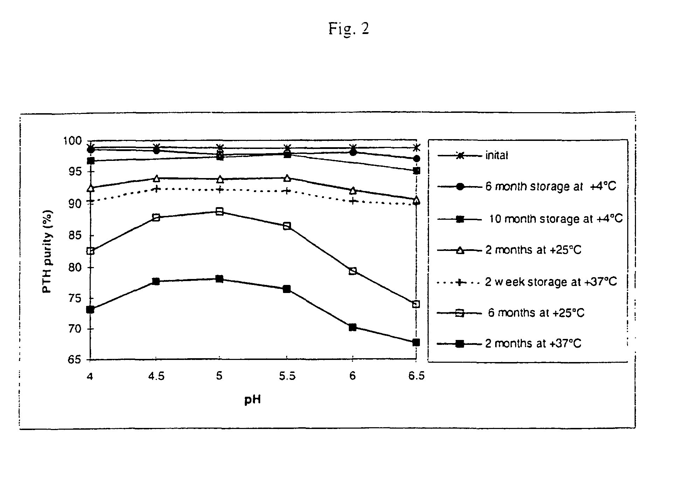

The results are shown in Table III and in Fig. 2. Degradation was accelerated at higher temperatures, as expected for most reactions. A rough calculation of this temperature effect showed a $Q_{10}{\approx}2$ in the temperature interval from +4°C to +25°C. and $Q_{10}{\approx}3$ for the temperature interval from +25°C to +37°C, at all pH values tested. This range of $Q_{10}$ values is consistent with other biochemical reactions, and justifies the use of the higher temperatures for accelerated degradation studies. The results indicate that the formulations at a pH from 4.5 to 5.5 have advantageous properties in terms of PTH stability. All formulations appeared as a clear colorless solution after storage. Furthermore, the formulations were stable at +4°C at a pH between 4 and 6.

EXAMPLE 3 - Effect of ionic strength on PTH stability

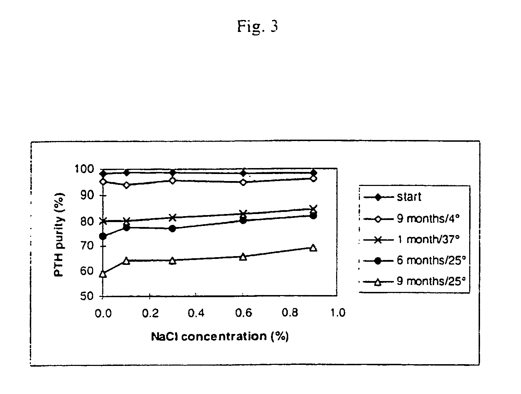

Formulations were prepared consisting of PTH (0.2 mg/ml), sodium citrate/citric acid (10 mM), pH 6. and mannitol (50 mg/ml). NaCl was added to the final concentrations indicated in Fig. 3. The formulations with added NaCl were hypertonic with respect to blood plasma. in addition to having an increased ionic strength. Aliquots (1 ml) were sealed in 2 ml glass vials under nitrogen and analyzed for purity of PTH by RP-HPLC.

The results, shown in Fig. 3. indicate that at higher temperatures, a small increase in stability was obtained with increased ionic strength. At +4°C, however, the formulations were not affected by increased ionic strength in terms of PTH stability. All formulations appeared as a clear colorless solution after storage.

EXAMPLE 4 - Effect of tonicity modifiers on PTH stability

Formulations were prepared comprising PTH (0.2 mg/ml) and sodium citrate/citric acid. pH 5.5 (10 mM), in the presence of a tonicity modifier (5% mannitol or 0.9% NaCl). Aliquots (0.7 ml) were sealed under nitrogen in 3 ml glass vials. After storage at the indicated temperatures. the vials were broken and the PTH formulation analyzed for purity. oxidized PTH (ox-M(8)-PTH and ox-M(18)-PTH), deamidated PTH (d16-PTH) and bioactivity using the adenylate cyclase assay.

The results, shown in Table IV, show that degradation was not affected by the tonicity modifier. Bioactivity of PTH was preserved at all temperatures studied. All formulations appeared as a clear colorless solution after storage. Formation of oxidized and deamidated PTH as well as decrease in PTH concentration was negligible after 18 months at +4°C.

EXAMPLE 5 - Effect of oxidative stress on PTH in prefilled syringes

Formulations were prepared comprising PTH (0.25 mg/ml), sodium citrate/citric acid. pH 5.5, (10 mM). and NaCl (9 mg/ml). Aliquots of 0.4 ml were filled into syringes fitted with a capped needle, and a rubber plunger was mounted making contact with the solution. The syringes where stored in either ambient air or in a protective nitrogen atmosphere, as indicated in Table V. The contents of the syringes were analyzed for PTH purity, oxidation and deamidation.

The results, shown in Table V, indicate that the presence of air does not appreciably affect neither oxidation, deamidation nor purity of PTH. Furthermore, the PTH formulations are stable at +4°C in a product container.

EXAMPLE 6 - Effect of preservatives on PTH stability

(a)

Formulations were prepared comprising PTH (1.3 mg/ml), sodium citrate/citric acid. pH 5.51(10 mM) and mannitol (50 mg/ml). Some formulations in addition comprised a preservative (3 mg/ml m-cresol or 1 mg/ml EDTA).
Aliquots (0.5 ml) of the formulations were sealed under nitrogen in 3 ml glass vials, and stored in a refrigerator (+2°C to +8°C) or at room temperature (+20°C to +25°C). After two weeks. the formulations were assayed for PTH purity and oxidized forms of PTH by RP-HPLC.
The results, shown in Table VI, indicate that the formulations were not affected by m-cresol or EDTA.

(b)

Formulations were prepared consisting of PTH (0.8 mg/ml), sodium citrate/citric acid, pH 5.5 (10 mM) and NaCl (9 mg/ml). The preservatives m-cresol or benzyl alcohol were added to some of the formulations at a concentration of 3 mg/ml or 10 mg/ml, respectively.
Aliquots of 0.5 ml were sealed under nitrogen in 3 ml glass vials. and stored in an inverted position, allowing the liquid to be in contact with the rubbers stopper. Following storage at +4°C, the formulations were assayed for PTH, oxidized PTH and deamidated PTH.
The results are shown in Table VII. Stability of PTH in the presence of m-cresol was comparable to that of control. In the presence of benzyl alcohol, the stability of PTH decreased, mostly attributable to the formation of M8 and M 18 oxidized forms of PTH. The concentration of PTH after storage was not affected by the presence of preservative (data not shown). Previous experiments have shown that the bioactivity of PTH is not affected by the presence of these preservatives. Furthermore, these preservatives gave a satisfactory protection against microbial growth after challenge, according to methods described in U.S. Pharmacopeia and European Pharmacopeia (data not shown). In the tested formulations. PTH is stable in the presence of efficient preservatives, thus allowing for the use of the formulation in a multidose product.

TABLE I

| PTH stability in lyophilized formulations | | | | | | | | |
|---|---|---|---|---|---|---|---|---|
| Formulation | IA | IB | IC | ID | IE | IF | IG | IH |
| PTH (mg/ml) | 0.072 | 0.1 | 0.2 | 0.5 | 1.5 | 3 | 5 | 5 |
| mannitol (mg/ml) | 50 | 35 | 35 | 35 | 50 | 50 | 50 | 50 |
| NaCl (mg/ml) | 0 | 0 | 0 | 0 | 1.2 | 2.4 | 0 | 4.5 |
| Temperature (°C) | 40 | 37 | 37 | 37 | 40 | 37 | 37 | 37 |
| pH | 5.5 | 6 | 6 | 6 | 5.5 | 5.5 | 5.5 | 5.5 |
| fill volume (ml) | 0.5 | 0.7 | 0.7 | 0.7 | 1 | 0.5 | 0.4 | 0.4 |
| Month | % PTH remaining from start | | | | | | | |
| 0 | 100 | 100 | 100 | 100 | 100 | 100 | 100 | 100 |
| 1 | n.d. | 99.28 | 98.08 | 99.28 | 99.60 | 99.60 | 99.76 | 99.86 |
| 2 | 98.48 | n.d. | n.d. | n.d. | 99.45 | 99.50 | 99.39 | 99.29 |
| 3 | 97.44 | 100.21 | 99.50 | 100.00 | 98.75 | 99.00 | n.d. | n.d. |
| 4 | n.d. | n.d. | n.d. | n.d. | n.d. | n.d. | n.d. | n.d. |
| 5 | n.d. | n.d. | n.d. | n.d. | n.d. | n.d. | 99.29 | 98.99 |
| 6 | n.d. | 92.97 | 94.96 | 98.46 | n.d. | 98.35 | n.d. | n.d. |
| 9 | n.d. | 89.87 | 91.53 | 95.80 | n.d. | n.d. | n.d. | n.d. |
| 12 | n.d. | 87.49 | 90.42 | 96.93 | n.d. | n.d. | n.d. | n.d. |

TABLE I (continued)

| PTH stability in lyophilized formulations | | | | | | | | |
|---|---|---|---|---|---|---|---|---|
| Formulation | IA | IB | IC | ID | IE | IF | IG | IH |
| | Degradation rate | | | | | | | |
| % per month | 0.84 | 1.15 | 0.84 | 0.32 | 0.39 | 0.27 | 0.13 | 0.21 |
| n.d. = not determined | | | | | | | | |

TABLE II

| PTH stability in liquid formulations | | | | | | | | |
|---|---|---|---|---|---|---|---|---|
| Formulation | 2A | 2B | 2C | 2D | 2E | 2F | 2G | 2H |
| PTH (mg/ml) | 0.2 | 0.2 | 0.2 | 0.2 | 0.2 | 0.25 | 0.5 | 0.78 |
| Mannitol (mg/ml) | 50 | 50 | 50 | 0 | 0 | 0 | 50 | 0 |
| NaCl (mg/ml) | 0 | 0 | 0 | 9 | 9 | 9 | 2 | 9 |
| Temperature (°C) | 4 | 4 | 4 | 4 | 4 | 4 | 4 | 4 |
| pH | 5 | 5.5 | 5.5 | 5 | 5.5 | 5.5 | 5.5 | 5.5 |
| Fill volume (ml) | 0.7 | 0.7 | 0.7 | 0.7 | 0.7 | 0.4 | 1.1 | I |
| Month | % PTH remaining from start | | | | | | | |
| 0 | 100 | 100 | 100 | 100 | 100 | 100 | 100 | 100 |
| 1 | n.d. | n.d. | n.d. | n.d. | n.d. | n.d. | 100.10 | n.d. |
| 2 | 101.03 | 100.20 | 100.10 | 101.23 | 101.44 | n.d. | n.d. | n.d. |
| 3 | n.d. | n.d. | n.d. | n.d. | n.d. | 100.15 | 99.20 | 99.60 |
| 4 | 100.62 | 99.29 | 99.80 | 100.31 | 97.42 | n.d. | n.d. | n.d. |
| 5 | n.d. | n.d. | n.d. | n.d. | n.d. | n.d. | n.d. | n.d. |
| 6 | 100.21 | 99.29 | 99.28 | 100.21 | 100.72 | 98.43 | 99.00 | 99.05 |
| 9 | 97.54 | 96.84 | 98.98 | 100.00 | 97.32 | n.d. | n.d. | n.d. |
| 12 | 97.43 | 96.22 | 96.12 | 96.71 | 96.39 | n.d. | n.d. | n.d. |
| 18 | 93.94 | 93.40 | 92.77 | 95.97 | 95.52 | n.d. | n.d. | n.d. |
| | Degradation rate | | | | | | | |
| % per month | 0.38 | 0.39 | 0.41 | 0.28 | 0.30 | 0.26 | 0.19 | 0.16 |
| n.d. = not determined | | | | | | | | |

TABLE III

| PTH stability at different pH values | | | | | | | | |
|---|---|---|---|---|---|---|---|---|
| | | pH | | | | | | |
| Storage | Temp. | 4 | 4.5 | 5 | 5.5 | 6 | 6.5 | |
| | | % PTH remaining from start | | | | | | |
| Inital | | 98.9 | 99 | 99 | 99 | 99 | 99 | |
| 2 weeks | +37°C | 90.5 | 92 | 92 | 92 | 90 | 90 | |
| 2 months | +25°C | 92.6 | 94 | 94 | 94 | 92 | 91 | |
| 2 months | +37°C | 73.2 | 78 | 78 | 76 | 70 | 68 | |

TABLE III   (continued)

| PTH stability at different pH values | | | | | | | |
|---|---|---|---|---|---|---|---|
| | | pH | | | | | |
| Storage | Temp. | 4 | 4.5 | 5 | 5.5 | 6 | 6.5 |
| | | % PTH remaining from start | | | | | |
| 6 months | +25°C | 82.6 | 88 | 89 | 87 | 79 | 74 |
| 6 months | +4°C | 98.5 | 98 | 98 | 98 | 98 | 97 |
| 10 months | +4°C | 96.8 | *n.d.* | 97 | 98 | *n.d.* | 95 |
| n.d. = not determined | | | | | | | |

TABLE IV

| PTH stability in the presence of tonicity modifiers | | | | | | | |
|---|---|---|---|---|---|---|---|
| | | PTH 1-84 Purity (%) | PTH 1-84 (µg/ml) | M8 (%) | M18 (%) | d16-PTH (%) | Bioactivity |
| Start | mannitol | 98.0 | 207 | 0.6 | 1.3 | n.d. | 0.64 |
| | NaCl | 96.9 | 209 | 1.0 | 1.7 | n.d. | 0.78 |
| 3 months +37° | mannitol | 65.4 | 137 | 7.9 | 11 | 24 | 1.15 |
| | NaCl | 70.8 | 156 | 8.1 | 9.4 | 21 | 0.89 |
| 6 months +25° | mannitol | 72.2 | 155 | 4.8 | 4.8 | 5.5 | 0.57 |
| | NaCl | 74.6 | 159 | 6.2 | 6 | 6.0 | 0.39 |
| 18 months +4° | mannitol | 91.5 | 204 | 1.2 | 2.1 | 0.4 | 0.4 |
| | NaCl | 92.6 | 200 | 1.5 | 2.4 | 0.6 | 0.27 |
| n.d.: not detected; M8 = ox-M(8)-PTH: M18 = ox-M(18)-PTH | | | | | | | |

TABLE V

| PTH stability in the presence of air | | | | | | |
|---|---|---|---|---|---|---|
| | | PTH 1-84 Purity (%) | PTH 1-84 (µg/ml) | M8 (%) | M18 (%) | d16-PTH (%) |
| Start | | 99.1 | 0.24 | 0.14 | 0.36 | 0.06 |
| 3 months +25°C | air | 84.2 | 0.22 | 3.9 | 4.6 | <d.l. |
| | nitrogen | 82.5 | 0.22 | 3.2 | 3.7 | <d.l. |
| 3 months +13°C | air | 96.7 | 0.23 | 0.84 | 1.0 | 0.59 |
| | nitrogen | n.d. | n.d. | n.d. | n.d. | n.d. |
| 4 months +4°C | air | 97.5 | n.d. | 0.77 | 1.0 | 0.32 |
| | nitrogen | 97.8 | n.d. | 0.59 | 0.88 | 0.36 |
| <d.l. = below detection level: n.d. = not determined; M8 = ox-M(8)-PTH; M18 = ox-M(18)-PTH | | | | | | |

TABLE VI

| PTH stability in the presence of preservatives | | | | |
|---|---|---|---|---|
| | | rhPTH(1-84) Purity (%) | M8 (%) | M18 (%) |
| Control | Refr. | 99.7 | 0.06 | 0.23 |
| | RT | 99.6 | 0.17 | 0.23 |
| m-cresol | Refr. | 99.6 | 0.14 | 0.23 |
| | RT | 99.3 | 0.27 | 0.39 |
| EDTA | Refr. | 99.7 | 0.11 | 0.24 |
| | RT | 99.4 | 0.27 | 0.34 |
| Refr. = Refrigerator: RT = Room temperature; M8 = ox-M(8)-PTH; M18 = ox-M(18)-PTH | | | | |

TABLE VII

| PTH stability in the presence of preservatives | | | | | |
|---|---|---|---|---|---|
| | Months | rhPTH(1-84) (%) | M8 (%) | M18 (%) | dPTH (%) |
| Control | 0 | 99.5 | 0.1 | 0.2 | 0.1 |
| | 3 | 99.1 | 0.2 | 0.3 | 0.2 |
| | 6 | 98.5 | 0.4 | 0.2 | 0.2 |
| m-cresol | 0 | 99.3 | 0.1 | 0.3 | <d.l. |
| | 3 | 99.2 | 0.1 | 0.3 | <d.l. |
| | 6 | 98.7 | 0.2 | 0.3 | <d.1. |
| benzyl alcohol | 0 | 99.0 | 0.2 | 0.5 | 0.1 |
| | 3 | 92.9 | 3.0 | 3.8 | 0.2 |
| | 6 | 92.4 | 3.1 | 3.8 | 0.2 |
| <d.1. = below detection level; M8 = ox-M(18)-PTH; M18 = ox-M(18)-PTH | | | | | |

**Claims**

1. A pharmaceutical formulation comprising human parathyroid hormone at a concentration of or above 0.3 mg/ml to 10 mg/ml; a pharmaceutically acceptable buffer having a pH from 4 to 6, and at least one tonicity modifier.

2. The formulation according to claim 1 wherein the said human parathyroid hormone is human recombinant parathyroid hormone.

3. The formulation according to claim 1 to 2 wherein the said human parathyroid hormone is a full-length parathyroid hormone.

4. The formulation according to any one of claims 1 to 3 wherein the concentration of the said human parathyroid hormone is from 0.3 mg/ml to 5 mg/ml.

5. The formulation according to claim 4 wherein the concentration of the said human parathyroid hormone is from 1 mg/ml to 3 mg/ml.

6. The formulation according to any one of claims 1 to 5 wherein the said pharmaceutically acceptable buffer is a citrate buffer at a concentration from 5 to 20 mM.

7. The formulation according to any one of daims 1 to 6 wherein the said pharmaceutically acceptable buffer has a pH between 5 and 6.

8. The formulation according to any one of claims 1 to 7 wherein the said tonicity modifier is sodium chloride and/or mannitol.

9. The formulation according to any one of claims 1 to 7 wherein the said tonicity modifier is sodium chloride.

10. The formulation according to any one of claims 1 to 7 wherein the said tonicity modifier is mannitol.

11. The formulation according to any one of claims 1 to 7 wherein the said tonicity modifier is sodium chloride and mannitol.

12. The formulation according to any one of claims 1 to 11 wherein the said human parathyroid hormone is a full-length parathyroid hormone.

13. The formulation according to any one of claims 1 to 12 comprising 1 to 3 mg/ml parathyroid hormone, 2 to 5 mg/ml NaCl, 20 to 50 mg/ml mannitol, 5 to 10 mM citrate buffer at a pH between 4 and 6, and optionally a preservative.

14. The formulation according to any one of claims 1 to 13 in liquid form.

15. The formulation according to any one of claims 1 to 13 in lyophilized form.

16. A process for the preparation of a pharmaceutical composition according to any one of claims 1 to 15, comprising dissolving human parathyroid hormone, to a concentration from 0.3 to 10 mg/ml, and at least one tonicity modifier, in a pharmaceutically acceptable buffer having a pH between 4 and 6.

17. A pharmaceutical formulation according to any one of claims 1 to 15 for use in the treatment or prevention of bone disorders.

18. A pharmaceutical formulation according to any one of claims 1 to 15 for use in the treatment or prevention of osteoporosis.

19. Use of parathyroid hormone at a concentration from 0.3 to 10 mg/ml, in the manufacture of a pharmaceutical formulation for the treatment or prevention of bone disorders, said pharmaceutical formulation in addition comprising a pharmaceutically acceptable buffer having a pH between 4 and 6, and at least one tonicity modifier.

20. The use according to claim 19 for the treatment or prevention of osteoporosis.

**Patentansprüche**

1. Pharmazeutische Formulierung umfassend humanes Parathyroidhormon bei einer Konzentration von oder über 0,3 mg/ml bis 10 mg/ml; ein pharmazeutisch verträglicher Puffer mit einem pH-Wert von 4 bis 6 und mindestens einen Tonizitätsmodifikator.

2. Formulierung gemäß Anspruch 1, wobei das humane Parathyroidhormon humanes rekombinantes Parathyroidhormon ist.

3. Formulierung gemäß Ansprüchen 1 bis 2, wobei das humane Parathyroidhormon ein Volllängen-Parathyroidhormon ist.

4. Formulierung gemäß irgendeinem der Ansprüche 1 bis 3, wobei die Konzentration des humanen Parathyroidhormons von 0,3 mg/ml bis 5 mg/ml ist.

5. Formulierung gemäß Anspruch 4, wobei die Konzentration des humanen Parathyroidhormons von 1 mg/ml bis 3 mg/ml ist.

**6.** Formulierung gemäß irgendeinem der Ansprüche 1 bis 5, wobei der pharmazeutisch verträgliche Puffer ein Zitratpuffer bei einer Konzentration von 5 bis 20 mM ist.

**7.** Formulierung gemäß irgendeinem der Ansprüche 1 bis 6, wobei der pharmazeutisch verträgliche Puffer einen pH-Wert zwischen 5 und 6 hat.

**8.** Formulierung gemäß irgendeinem der Ansprüche 1 bis 7, wobei der Tonizitätsmodifikator Natriumchlorid und/oder Mannitol ist.

**9.** Formulierung gemäß irgendeinem der Ansprüche 1 bis 7, wobei der Tonizitätsmodifikator Natriumchlorid ist.

**10.** Formulierung gemäß irgendeinem der Ansprüche 1 bis 7, wobei der Tonizitätsmodifikator Mannitol ist.

**11.** Formulierung gemäß irgendeinem der Ansprüche 1 bis 7, wobei der Tonizitätsmodifikator Natriumchlorid und Mannitol ist.

**12.** Formulierung gemäß irgendeinem der Ansprüche 1 bis 11, wobei das humane Parathyroidhormon ein Volllängen-Parathyroidhormon ist.

**13.** Formulierung gemäß irgendeinem der Ansprüche 1 bis 12, umfassend 1 bis 3 mg/ml Parathyroidhormon, 2 bis 5 mg/ml NaCl, 20 bis 50 mg/ml Mannitol, 5 bis 10 mM Zitratpuffer bei einem pH-Wert zwischen 4 und 6, und gegebenenfalls ein Konservierungsmittel.

**14.** Formulierung gemäß irgendeinem der Ansprüche 1 bis 13 in flüssiger Form.

**15.** Formulierung gemäß irgendeinem der Ansprüche 1 bis 13 in lyophilisierter Form.

**16.** Verfahren zur Herstellung einer pharmazeutischen Zusammensetzung gemäß irgendeinem der Ansprüche 1 bis 15, umfassend das Lösen von humanem Parathyroidhormon auf eine Konzentration von 0,3 bis 10 mg/ml, und von mindestens einem Tonizitätsmodifikator in einem pharmazeutisch verträglichen Puffer mit einem pH-Wert zwischen 4 und 6.

**17.** Pharmazeutische Formulierung gemäß irgendeinem der Ansprüche 1 bis 15 zur Verwendung bei der Behandlung oder Vorbeugung von Knochenstörungen.

**18.** Pharmazeutische Formulierung gemäß irgendeinem der Ansprüche 1 bis 15 zur Verwendung bei der Behandlung oder Vorbeugung von Osteoporose.

**19.** Verwendung von Parathyroidhormon bei einer Konzentration von 0,3 bis 10 mg/ml bei der Herstellung einer pharmazeutischen Formulierung für die Behandlung oder Vorbeugung von Knochenstörungen, wobei die pharmazeutische Formulierung zusätzlich einen pharmazeutisch verträglichen Puffer mit einem pH-Wert zwischen 4 und 6, und mindestens einen Tonizitätsmodifikator umfasst.

**20.** Verwendung gemäß Anspruch 19 für die Behandlung oder Vorbeugung von Osteoporose.

**Revendications**

**1.** Formulation pharmaceutique comprenant l'hormone parathyroïdienne humaine à une concentration égale ou supérieure à 0,3 mg/ml jusqu'à 10 mg/ml, un tampon pharmaceutiquement acceptable ayant un pH de 4 à 6, et au moins un modificateur de tonicité.

**2.** Formulation selon la revendication 1 dans laquelle ladite hormone parathyroïdienne humaine est une hormone parathyroïdienne humaine recombinante.

**3.** Formulation selon la revendication 1 ou 2 dans laquelle ladite hormone parathyroidienne humaine est une hormone parathyroïdienne complète.

**4.** Formulation selon l'une quelconque des revendications 1 à 3 dans laquelle la concentration de ladite hormone parathyroïdienne humaine est de 0,3 mg/ml à 5 mg/ml.

**5.** Formulation selon la revendication 4 dans laquelle la concentration de ladite hormone parathyroïdienne humaine est de 1 mg/ml à 3 mg/ml.

**6.** Formulation selon l'une quelconque des revendications 1 à 5 dans laquelle ledit tampon pharmaceutiquement acceptable est un tampon citrate à une concentration de 5 à 20 mM.

**7.** Formulation selon l'une quelconque des revendications 1 à 6 dans laquelle ledit tampon pharmaceutiquement acceptable a un pH entre 5 et 6.

**8.** Formulation selon l'une quelconque des revendications 1 à 7 dans laquelle ledit modificateur de tonicité est le chlorure de sodium et/ou le mannitol.

**9.** Formulation selon l'une quelconque des revendications 1 à 7 dans laquelle ledit modificateur de tonicité est le chlorure de sodium.

**10.** Formulation selon l'une quelconque des revendications 1 à 7 dans laquelle ledit modificateur de tonicité est le mannitol.

**11.** Formulation selon l'une quelconque des revendications 1 à 7 dans laquelle ledit modificateur de tonicité est le chlorure de sodium et le mannitol.

**12.** Formulation selon l'une quelconque des revendications 1 à 11 dans laquelle ladite hormone parathyroïdienne humaine est une hormone parathyroïdienne complète.

**13.** Formulation selon l'une quelconque des revendications 1 à 12 comprenant 1 à 3 mg/ml d'hormone parathyroïdienne, 2 à 5 mg/ml de NaCl, 20 à 50 mg/ml de mannitol, 5 à 10 mM de tampon citrate à un pH entre 4 et 6, et éventuellement un conservateur.

**14.** Formulation selon l'une quelconque des revendications 1 à 13 sous forme liquide.

**15.** Formulation selon l'une quelconque des revendications 1 à 13 sous forme lyophilisée.

**16.** Procédé pour la préparation d'une composition pharmaceutique selon l'une quelconque des revendications 1 à 15, consistant à dissoudre l'hormone parathyroïdienne humaine à une concentration de 0,3 à 10 mg/ml, et au moins un modificateur de tonicité, dans un tampon pharmaceutiquement acceptable ayant un pH entre 4 et 6.

**17.** Formulation pharmaceutique selon l'une quelconque des revendications 1 à 15 à utiliser dans le traitement ou la prévention des troubles osseux.

**18.** Formulation pharmaceutique selon l'une quelconque des revendications 1 à 15 à utiliser dans le traitement ou la prévention de l'ostéoporose.

**19.** Utilisation d'hormone parathyroïdienne à une concentration de 0,3 à 10 mg/ml dans la fabrication d'une formulation pharmaceutique destinée au traitement ou à la prévention de troubles osseux, ladite formulation pharmaceutique comprenant en outre un tampon pharmaceutiquement acceptable ayant un pH entre 4 et 6, et au moins un modificateur de tonicité.

**20.** Utilisation selon la revendication 19 pour le traitement ou la prévention de l'ostéoporose.

Fig. 1

PTH degradation rate *Vs* PTH concentration

Fig. 2

Fig. 3